# EUROPÄISCHE PATENTSCHRIFT

(11) **EP 1 684 653 B1**
(45) Veröffentlichungstag und Bekanntmachung des Hinweises auf die Patenterteilung: **23.06.2010**
(21) Anmeldenummer: 04791021.1
(22) Anmeldetag: 29.10.2004
(51) Int. Cl.: A61B 18/00

(54) **INSTRUMENT FÜR DIE PLASMA-KOAGULATION**
INSTRUMENT FOR PLASMA COAGULATION
INSTRUMENT DE COAGULATION AU PLASMA

(30) Priorität: 04.11.2003 DE 10351370
(43) Veröffentlichungstag der Anmeldung: 02.08.2006
(73) Patentinhaber: Erbe Elektromedizin GmbH, 72072 Tübingen (DE)
(72) Erfinder: SCHNITZLER, Uwe, 72074 Tübingen (DE)
(74) Vertreter: Bohnenberger, Johannes
(86) Internationale Anmeldenummer: PCT/EP2004/012260
(87) Internationale Veröffentlichungsnummer: WO 2005/046495

(56) Entgegenhaltungen:
- EP-A- 0 740 926
- EP-A- 1 293 169
- DE-A- 10 030 111
- US-A- 5 320 621

## Beschreibung

Die Erfindung betrifft ein Instrument für die Plasma-Koagulation nach dem Oberbegriff des Patentanspruches 1.

Ein derartiges Instrument ist aus der DE 100 30 111 A1 bekannt.

Ein ähnliches Instrument ist beispielsweise aus der EP-1293170 A1 bekannt und wird nachfolgend anhand der beiliegenden Fig. 3 erläutert.

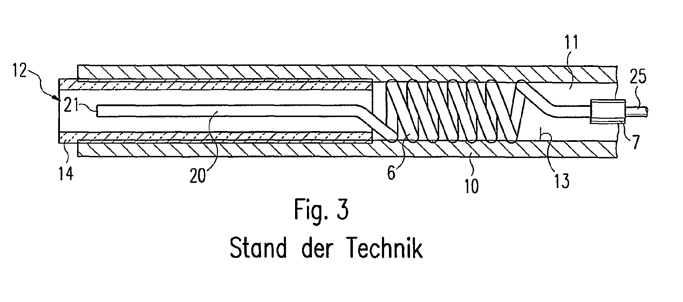

In Fig. 3 ist eine Sonde für die Plasma-Koagulation und zwar mittels Argon (APC) in einem Längsschnitt desjenigen Endabschnittes gezeigt, der bei einer Operation aus der Mündung des Arbeitskanals eines Endoskopes hervorsteht. Durch das Lumen 11 eines Sondenkörpers 10 strömt Argon-Gas und tritt aus einer Mündung 12 des Sondenkörpers 10 aus.

Im Lumen 11 des Sondenkörpers 10 befindet sich eine Zündelektrode 20, die an ihrem, einer Spitze 21 abgewandten Ende eine Wendel 6 aufweist, welche derart gewickelt ist, dass der Außenumfang der Wendel 6 fest an einer Rohrwand 13 des Sondenkörpers 10 anliegt bzw. mit dieser fest verbunden ist. Das der Zündelektrode 20 gegenüberliegende Ende der Wendel 6 ist über ein Crimp-Röhrchen 7 mit einem Stromleiter 25 zur Zuleitung eines Koagulationsstroms verbunden. Die Zündelektrode 20 befindet sich im Inneren eines aus Keramik gefertigten Röhrchens 14, welches ebenfalls in die Rohrwand 13 fest eingepresst ist. Die Anordnung ist hierbei derart getroffen, dass die Zündelektrode 20 mit ihrer Spitze 21 um einen vorbestimmten Betrag zurückversetzt ist von der Mündung 12 des Sondenkörpers 10 bzw. des darin befindlichen Röhrchens 14. Weiterhin ist die Zündelektrode 20 exakt koaxial innerhalb des Röhrchens 14 angeordnet, so dass symmetrische Verhältnisse entstehen, wenn das an der Zündelektrode 20 bzw. deren Spitze 21 vorbei und durch die Mündung 12 ausströmende Argon durch den Koagulationsstrom ionisiert wird und somit ein Plasma in der bekannten Weise entsteht, welches zu behandelndes Gewebe koaguliert.

Bei der aus der EP-1293170 A1 bekannten Anordnung tritt insbesondere hinsichtlich der Fertigung aber auch hinsichtlich des Betriebes, insbesondere Dauerbetriebes eine Vielzahl von Problemen auf.

Zunächst ist es schwierig, die Zündelektrode 20 mit einer exakt gefertigten Wendel 6 derart zu versehen, dass bei Einpressen der Wendel 6 in den Sondenkörper 10 die Zündelektrode 20 und insbesondere die Spitze 21 exakt mittig bzw. koaxial zum Röhrchen 14 zu liegen kommt. Weiterhin tritt ein Problem dahin gehend auf, dass der Abstand der Spitze 21 zur Mündung 12 des Röhrchens 14 abhängt von der Einpresstiefe der Wendel 6. Eine direkte Anlage der Wendel 6 am Hinterende des Röhrchens 14 kann diesem Problem auch nicht abhelfen, da ein zu festes Anpressen zu einer Lage- bzw. Winkelveränderung der Zündelektrode 20 führen würde.

Die Verbindung zwischen dem Stromleiter 25 und der Elektrode 20 über das Crimp-Röhrchen 7 ist arbeitsaufwändig, wobei immer zu bedenken ist, dass das Lumen 11 einen sehr geringen Durchmesser, bei Sonden einen Durchmesser im unteren mm-Bereich und sogar noch darunter hat.

Ein weiteres Problem bei dem aus der EP-1293170 A1 bekannten Instrument liegt darin, dass insbesondere durch die Wendel 6 und den Übergang von ihr zur geradegestreckten Zündelektrode 20 zum einen eine Verengung des vom Edelgas durchströmten Raumes bewirkt wird, wobei weiterhin im Übergang zwischen der Wendel 6 und dem Innenraum des Röhrchens eine Unsymmetrie vorliegt, was zu ungleichmäßigen Gasströmungsverhältnissen innerhalb des Röhrchens 14 führt. Alle diese Unsymmetrien und Verengungen führen zu einer Störung des Plasma-Aufbaus und insbesondere auch zu einer schlechten Reproduzierbarkeit in Serie gefertigter Instrumente hinsichtlich deren Zündverhaltens.

Auch in thermischer Hinsicht wirft der aus der EP-1293170 A1 bekannte Stand der Technik Probleme auf. Das Plasma führt nämlich zu einem Aufheizen und sogar Abbrennen der Zündelektrode 20, so dass sich das Brennverhalten, insbesondere aber das Zündverhalten des Instruments während einer Anwendung ändert, was wiederum ein Nachjustieren des Koagulationsstroms (bzw. der angelegten HF-Spannungsamplitude) notwendig macht.

Der Erfindung liegt die Aufgabe zu Grunde, ein Instrument der genannten Art dahin gehend weiterzubilden, dass bei vereinfachtem Aufbau bzw. Herstellungsverfahren eine verbesserte Reproduzierbarkeit des Zünd- und Brennverhaltens gewährleistet wird.

Diese Aufgabe wird bei einem Instrument der genannten Art dadurch gelöst, dass die Befestigungseinrichtung ein Blech, ein Plättchen oder dergleichen flächigen Körper umfasst, der mit Längsrändern an der Rohrwand, das Lumen im Wesentlichen diametral durchquerend fixiert ist und an dem die Zündelektrode befestigt ist.

Durch diese Konstruktion wird zunächst die Fixierung der Zündelektrode innerhalb des Sondenkörpers erleichtert, da keine Wendel mehr gewickelt werden muss. Dadurch, dass der Körper flächig ausgebildet ist, kann das vorbeiströmende Edelgas den Körper und damit die an ihn befestigte Zündelektrode kühlen. Dadurch, dass der flächige Körper an seinen beiden Rändern mit der Rohrwand in Verbindung steht, ist ein symmetrischer Aufbau insbesondere im Übergang zwischen dem flächigen Körper, also dem die Zündelektrode haltenden Abschnitt und der Zündelektrode selbst gewährleistet, was wiederum symmetrische Strömungsverhältnisse und insbesondere auch keine wesentlichen Veränderungen des Lumens des Sondenkörpers mit sich bringt. Das Einpressen des flächigen Körpers in das Lumen des Sondenkörpers ist sehr einfach, da er in sich steif ist und nicht - wie eine Wendel - während des Einpressens verspannt wird, so dass diese Spannungen im späteren Gebrauch sich ausgleichen und dadurch Verschiebungen auftreten könnten.

Der Stromleiter wird bei einer Ausführungsform der Erfindung mit der Zündelektrode einstückig verbunden. Es kann hier also ein Wolfram-Draht verwendet werden, der durchgängig von der Zündelektrode über das Befestigungsplättchen bzw. den flächigen Körper bis zum Stecker führt, über welchen der Stromleiter dann an das elektrochirurgische Gerät angeschlossen wird.

Alternativ wird der Stromleiter über den flächigen Körper mit der Zündelektrode verbunden, so dass der flächige Körper, also die Halterung der Zündelektrode im Sondenkörper, das bei der bekannten Anordnung notwendige Crimp-Röhrchen ersetzt.

Die Zündelektrode und/oder der Stromleiter sind vorzugsweise am flächigen Körper angeschweißt, also über eine Verbindungsart fixiert, die besonders sicher und einfach herzustellen ist. Besonders eignet sich hierzu eine Widerstandsschweißung über Schweißpunkte.

Im Bereich der Mündung ist erfindungsgemäß ein Röhrchen aus Keramik oder dergleichen hochtemperaturfestem Material (wie an sich bekannt) in das Lumen eingesetzt, wobei der flächige Körper an einer von der Mündung abgewandten Seite des Röhrchens angeordnet ist. Dadurch wird ein höhere Standfestigkeit der Anordnung erzielt. Weiterhin ist ein Anschlag zwischen dem flächigen Körper bzw. ein Anschlag von Abschnitten seines Vorderendes am Röhrchen vorgesehen. Auf diese Weise ist eine exakt und eindeutig reproduzierbare geometrische Zuordnung zwischen der Halterung der Zündelektrode (also dem flächigen Körper) und dem, die Mündung definierenden Keramikröhrchen in einfacher Weise sichergestellt.

Der flächige Körper weist vorzugsweise an einem, der Mündung zugewandten Vorderrand einen konkaven Ausschnitt auf. Dadurch ist einerseits eine hinreichend große Verbindungsfläche des flächigen Körpers mit der Rohrwand sichergestellt, andererseits wird das Lumen über einen längeren Abschnitt der Zündelektrode frei von verengenden Materialabschnitten gehalten. Dies ist insbesondere dann von großem Vorteil, wenn ein Keramikröhrchen verwendet wird, an welchem der flächige Körper anschlägt. In diesem Fall befindet sich also die Ausnehmung in Strömungsrichtung es Edelgases gesehen vor dem Keramikröhrchen, so dass besonders störungsfreie und symmetrische Strömungsverhältnisse innerhalb des Röhrchens sichergestellt werden.

Nachfolgend werden bevorzugte Ausführungsformen der Erfindung anhand von Abbildungen näher erläutert. Hierbei zeigen
- Fig. 1: eine Ausführungsform, die nicht zur Erfindung gelöst,
- Fig. 2: eine bevorzugte Ausführungsform der Erfindung und
- Fig. 3: ein Instrument nach dem Stand der Technik.

In der nachfolgenden Beschreibung werden für gleiche und gleich wirkende Teile dieselben Bezugsziffern verwendet, wobei insbesondere diejenigen Teile nicht nochmals gesondert erläutert werden, die bereits im Zusammenhang mit Fig. 3 oben schon erläutert wurden.

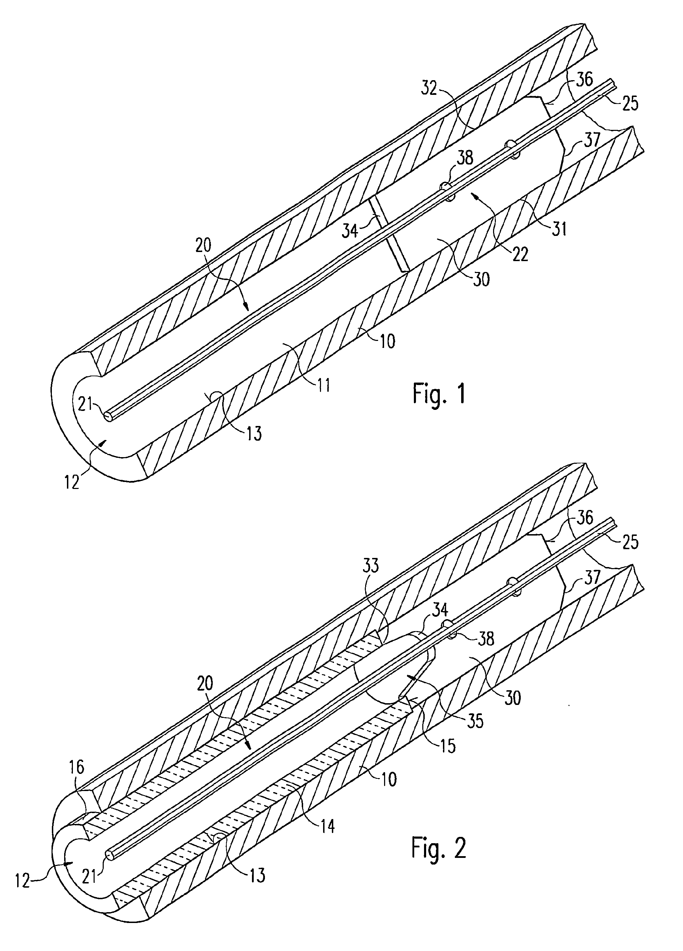

Wie aus Fig. 1 ersichtlich ist zur Befestigung der Zündelektrode 20 innerhalb eines Befestigungsbereiches 22 ein flächiger Körper 30 vorgesehen, der mit Längsrändern 31 und 32 in das Lumen 11 des Sondenkörpers 20 von dessen Mündung 12 her derart eingepresst ist, dass diese Längsränder 31, 32, und damit der gesamte flächige Körper 30 in festem Kontakt mit der Rohrwand 30 des Sondenkörpers 10 stehen. Um das Einpressen zu erleichtern, weist der flächige Körper 30 an seinem, der Mündung 12 gegenüberliegenden Ende Anfasungen 37 an seinem Hinterrand 36 auf.

Die Befestigung der Zündelektrode 20, die mit dem Stromleiter 25 als einstückig verbundener (Wolfram-) Draht ausgeführt ist, mit dem flächigen Körper 30 ist über Schweißpunkte 38 bewerkstelligt, welche vorzugsweise durch Punkt-Widerstandsschweißen erzeugt sind.

Bei der hier gezeigten Anordnung ist leicht vorstellbar, dass eine exakte, konzentrische Anordnung der Zündelektrode 20 und insbesondere deren Spitze 21 dadurch sehr leicht herstellbar ist, dass der vorzugsweise aus Stahlblech oder Wolframblech gefertigte flächige Körper 30 exakt gefertigt und die Zündelektrode 20 samt Stromleiter 25 exakt in seiner Mitte angepunktet sind. Die konzentrische Ausrichtung des flächigen Körpers 30 innerhalb des Lumens 11 geschieht sozusagen von selbst aufgrund der exakt symmetrischen Ausbildung des Lumens 11 bzw. der Rohrwand 13.

Die Ausführungsform nach Fig. 2 unterscheidet sich von der nach Fig. 1 dadurch, dass im Bereich des Endes des Sondenkörpers (in an sich bekannter Weise) ein Keramikröhrchen 14 eingesetzt ist. Der flächige Körper 30 weist an seinem, der Mündung 12 zugewandten Vorderrand 34 einen Ausschnitt 35 auf, der in Anschläge 33 an den beiden Längsrändern 31, 32 übergeht. Die Anschläge 33 sind derart dimensioniert, dass sie im Wesentlichen der Dicke des Röhrchens 14 entsprechen. Durch diese Ausgestaltung ist einerseits gewährleistet, dass exakte und einwandfrei reproduzierbare geometrische Verhältnisse zwischen dem flächigen Körper 30 samt der auf ihm befestigten Zündelektrode 20 und dem Röhrchen 14 gewährleistet sind, welches die Mündung 12 definiert. Der Abstand, den die Spitze 21 der Zündelektrode 20 von der Mündung 12 hat, hängt nicht vom Vorgang des Fixierens der Zündelektrode 20 innerhalb des Sondenkörpers 10 ab sondern ausschließlich von der mit entsprechenden Werkzeugen sicherzustellenden Präzision der Fertigung von flächigem Körper 30 und Befestigung der Zündelektrode 20 auf diesem ab. Insofern stellt also der Kontakt zwischen dem Anschlag 33 des flächigen Körpers 30 und dem Hinterrand 15 des Röhrchens 14 ein wesentliches Justierungskriterium dar.

Neben den oben beschriebenen Vorteilen des flächigen Körpers 30 insbesondere in Bezug auf die Kühlung der Zündelektrode 20 (wodurch ein besseres Abbrandverhalten erzielt wird) wird durch den Ausschnitt 35 gewährleistet, dass das Innere des Röhrchens 14 auch in seinen Anfangsbereichen (in Strömungsrichtung des Gases gesehen) frei bleibt.

### Bezugszeichenliste

- 6: Wendel
- 7: Crimp-Röhrchen
- 10: Sondenkörper
- 11: Lumen
- 12: Mündung
- 13: Rohrwand
- 14: Röhrchen
- 15: Röhrchen-Hinterwand
- 20: Zündelektrode
- 21: Spitze
- 22: Befestigungsbereich
- 25: Stromleiter
- 30: Flächiger Körper
- 31: Längsrand
- 32: Längsrand
- 33: Anschlag
- 34: Vorderrand
- 35: Ausschnitt
- 36: Hinterrand
- 37: Anfasung
- 38: Schweißpunkt

## Patentansprüche

1. Instrument für die Plasma-Koagulation (APC), umfassend
einen rohrförmigen Sondenkörper (10) zum Hindurchleiten von Edelgas durch ein von einer Rohrwand (13) gebildetes Lumen (11) des Sondenkörpers (10),
eine Zündelektrode (20) innerhalb des Lumens (11) und im Bereich einer Mündung (12) des Sondenkörpers (10),
einen Stromleiter (25) zum Zuleiten eines Koagulationsstroms zur Zündelektrode (20),
Befestigungseinrichtungen (30) zum Befestigen der Zündelektrode (20) in einer definierten Position im Sondenkörper (10),
wobei
die Befestigungseinrichtung einen flächigen Körper (30), insbesondere ein Blech oder ein Plättchen, umfasst, der mit Längsrändern (31, 32) an der Rohrwand (13), das Lumen (11) im Wesentlichen diametral durchquerend fixiert ist und an dem die Zündelektrode (20) befestigt ist,
**dadurch gekennzeichnet, dass**
im Bereich der Mündung (12) ein Röhrchen (14) aus hochtemperaturfestem Material, insbesondere aus Keramik, in das Lumen (11) eingesetzt ist und der flächige Körper (30) an einer von der Mündung (12) abgewandten Seite (15) des Röhrchens (14) angeordnet ist und mit Abschnitten (33) seines Vorderrandes (34) am Röhrchen (14) anschlägt.

2. Instrument nach Anspruch 1,
**dadurch gekennzeichnet, dass**
der Stromleiter (25) mit der Zündelektrode (20) einstückig verbunden ist.

3. Instrument nach Anspruch 1,
**dadurch gekennzeichnet, dass**
der Stromleiter (25) über den flächigen Körper (30) mit der Zündelektrode (20) verbunden ist.

4. Instrument nach einem der vorhergehenden Ansprüche,
**dadurch gekennzeichnet, dass**
die Zündelektrode (20) und/oder der Stromleiter (25) am flächigen Körper (30) angeschweißt sind.

5. Instrument nach Anspruch 4,
**dadurch gekennzeichnet, dass**
die Anschweißung (38) über Widerstandsschweißung punktförmig ausgebildet ist.

6. Instrument nach einem der vorhergehenden Ansprüche,
**dadurch gekennzeichnet, dass**
der flächige Körper (30) an seinem, der Mündung (12) zugewandten Vorderrand (34) einen konkaven Ausschnitt (35) aufweist.

## Claims

1. Instrument for plasma coagulation (APC), comprising
a tubular probe body (10) for conducting inert gas through a lumen (11) of the probe body (10), which lumen is formed by a tube wall (13),
an ignition electrode (20) inside the lumen (11) and in the region of an orifice (12) of the probe body (10),
a current conductor (25) for supplying a coagulation current to the ignition electrode (20),
fixing devices (30) for fixing the ignition electrode (20) in a defined position in the probe body (10),
wherein the fixing device comprises a planar body (30), especially a metal sheet or a plate, which is fixed to the tube wall (13) by longitudinal edges (31, 32) so as to extend substantially diametrically across the lumen (11) and to which the ignition electrode (20) is fixed,
**characterized in that**
a tube (14) made of high-temperature-resistant material, especially of ceramics, is inserted in the lumen (11) in the region of the orifice (12), and the planar body (30) is arranged at the end (15) of the tube (14) remote from the orifice (12) and abuts the tube (14) with portions (33) of its forward edge (34).

2. Instrument according to claim 1,
**characterized in that**
the current conductor (25) is integrally joined to the ignition electrode (20).

3. Instrument according to claim 1,
**characterized in that**
the current conductor (25) is joined to the ignition electrode (20) by way of the planar body (30).

4. Instrument according to any one of the preceding claims,
**characterized in that**
the ignition electrode (20) and/or the current conductor (25) is/are welded to the planar body (30).

5. Instrument according to claim 4,
**characterized in that**
the weld (38) is a spot weld made by resistance welding.

6. Instrument according to any one of the preceding claims,
**characterized in that**
the planar body (30) has a concave recess (35) in its forward edge (34) facing the orifice (12).

## Revendications

1. Instrument de coagulation au plasma (APC) comprenant un corps de sonde (10) tubulaire destiné à conduire un gaz rare à travers une lumière (11) du corps de sonde (10) définie par une paroi tubulaire (13),
une électrode d'amorçage (20) à l'intérieur de la lumière (11) et, dans la zone de l'embouchure (12) du corps de sonde (10),
un conducteur électrique (25) destiné à conduire un courant de coagulation vers l'électrode d'amorçage (20),
des moyens de fixation (30) destinés à fixer l'électrode d'amorçage (20) selon une position définie dans le corps de sonde (10),
les moyens de fixation comprenant un corps plan (30) notamment une plaque ou une plaquette qui est fixée à la paroi tubulaire (13) par ses bords longitudinaux (31, 32) et en traversant sensiblement diamétralement la lumière (11) et à laquelle est fixée l'électrode d'amorçage (20),
**caractérisé en ce que,**
dans la zone de l'embouchure (12), un petit tube (14) en matériau résistant aux hautes températures, notamment en céramique, est mis en place dans la lumière (11), et **en ce que** le corps plan (30) est agencé sur un côté (15) du petit tube (14) qui est opposé à l'embouchure (12) et vient en butée contre le petit tube (14) par des portions (33) de son bord avant (34).

2. Instrument selon la revendication 1, **caractérisé en ce que** le conducteur électrique (25) est raccordé monobloc à l'électrode d'amorçage (20).

3. Instrument selon la revendication 1, **caractérisé en ce que** le conducteur électrique (25) est raccordé à l'électrode d'amorçage (20) par l'intermédiaire du corps plan (30).

4. Instrument selon l'une quelconque des revendications précédentes, **caractérisé en ce que** l'électrode d'amorçage (20) et/ou le conducteur électrique (25) sont rapportés par soudage sur le corps plan (30).

5. Instrument selon la revendication 4, **caractérisé en ce que** la soudure (38) est obtenue par soudage par points par résistance.

6. Instrument selon l'une quelconque des revendications précédentes, **caractérisé en ce que** le corps plan (30) présente une échancrure concave (35) au niveau de son bord avant (34) en regard de l'embouchure (12).
